# EUROPÄISCHE PATENTSCHRIFT

(11) **EP 2 114 385 B1**
(45) Veröffentlichungstag und Bekanntmachung des Hinweises auf die Patenterteilung: **18.03.2020**
(21) Anmeldenummer: 08715656.8
(22) Anmeldetag: 19.01.2008
(51) Int. Cl.: A61K 9/70, A61K 31/135

(54) **TRANSDERMALE THERAPEUTISCHES SYSTEM ZUR VERABREICHUNG WASSERLÖSLICHER WIRKSTOFFE**
TRANSDERMAL THERAPEUTIC SYSTEM FOR ADMINISTERING WATER-SOLUBLE ACTIVE INGREDIENTS
SYSTÈME THÉRAPEUTIQUE TRANSDERMIQUE POUR L'ADMINISTRATION D'AGENTS ACTIFS SOLUBLES DANS L'EAU

(30) Priorität: 08.02.2007 DE 102007006244
(43) Veröffentlichungstag der Anmeldung: 11.11.2009
(73) Patentinhaber: LTS LOHMANN Therapie-Systeme AG, 56626 Andernach (DE)
(72) Erfinder: ASMUSSEN, Bodo, 56170 Bendorf (DE); HORSTMANN, Michael, 56564 Neuwied (DE); SCHMITZ, Christoph, 56598 Rheinbrohl (DE); SAMETI, Mohammad, 53177 Bonn (DE); PRZYBYLLA, Yves-Thorsten, 56645 Nickenich (DE); PRACHT, Rolf, 56203 Höhr-Grenzhausen (DE)
(74) Vertreter: Plate, Jürgen
(86) Internationale Anmeldenummer: PCT/EP2008/000392
(87) Internationale Veröffentlichungsnummer: WO 2008/095597

(56) Entgegenhaltungen:
- WO-A-2004/060447
- WO-A-2005/037157
- US-A1- 2003 147 943
- US-A1- 2004 247 657
- US-A1- 2005 074 487
- US-A1- 2005 244 485
- US-A1- 2006 093 659

## Beschreibung

Transdermale Therapeutische Systeme sind für viele Wirkstoffe seit einer Reihe von Jahre im Markt etabliert. Solche Applikationsformen erlauben den Durchschnitt von hautpermeationsfähigen pharmazeutischen Wirkstoffen durch die gesunde menschliche Haut zum Zwecke der Erzielung systemischer therapeutischer Wirkungen. In der Regel beruhen solche pharmazeutische Wirkstoffpflaster auf der Basis von so genannten Matrix- oder Reservoir/Membranpflastern, welche den Wirkstoff in gelöster oder kristalliner Form in einem überwiegend lipophilen Polymer eingebettet enthalten. Zahlreiche Technologien basieren auch auf dem Zusatz überwiegend lipidlöslicher Hilfsstoffe, die teilweise eine verbesserte Klebkraft oder Diffusivität des Wirkstoffs bewirken sollen, andererseits auch die Verstärkung der Resorptionswirkung der Haut selbst zum Ziele haben.

Die Abgabe von vorwiegend hydrophilen Wirkstoffen an die menschliche Haut, welche nur beschränkte Löslichkeit in lipophilen Medien aufweisen, wurde bisher weniger intensiv erforscht. Es sind zwar Hydrogel-Systeme bekannt, deren klebende, der Haut zugewandte Matrix überwiegend aus Wasser besteht und daher die Verwendung gut wasserlöslicher Wirkstoffe zuläßt. Nachteilig an solchen Systemen ist jedoch, dass insbesondere hochmolekulare pharmazeutische Wirkstoffe durch das vorhandene polymere Gelgerüst an ihrem Diffusionsverhalten stark gehindert werden, bevor sie die Haut erreichen.

In US Pat 5 707 641 wird eine pharmazeutische Formulierung, bestehend aus einer wässrigen Emulsion oder Dispersion, insbesondere für die transdermale Anwendung beschrieben, welche zusätzlich zur wässrigen Phase als Wirkstoff ein pharmazeutisch aktives Protein oder Polypeptid, einen Emulgator und eine ölige Phase enthält. Die Patentschrift offenbart ferner eine Matrix für die transdermale Administration der genannten Formulierung, bestehend aus einem porösen, absorbierenden, und monolaminaren festen Material, das die genannte Formulierung absorbiert enthält. Die Matrix kann ferner mit einer flexiblen atmungsfähigen (d.h. nicht okklusiven) Rückschicht versehen sein.

In EP 0 412 869 B1 wird ein Kompositfilm zur lokalen Behandlung der Hautoberfläche vorgeschlagen, welcher eine Okklusivschicht und eine Reservoirschicht umfasst. Die letztere wird aus einer, aus einem Silikonpolymer bestehenden Matrix gebildet und enthält in inneren Einschlüssen eine wässrige Gelschicht mit einem pharmazeutischen Wirkstoff. Zur Verstärkung kann die Reservoirschicht eine Einlage aus perforiertem Faservlies enthalten, wobei diese Einlage jedoch nicht mit dem wirkstoffhaltigen Gel in Berührung tritt. Aus der Beschreibung und den Ansprüchen wird deutlich, dass dieser Kompositfilm nur zur lokalen Behandlung der Haut geeignet ist, da keine Maßnahmen angegeben werden, welche die für eine systemisch transdermale Applikation erforderliche kontrollierte Freisetzung des Wirkstoffs gewährleisten.

Der zuletzt genannte Mangel liegt auch vor bei Vorschlägen zur Applikation von wirkstoffhaltigen, wässrigen Formulierungen, gemäß der indischen Patentschrift IN 187032 sowie der DE 42 23 004 A1, worin die Applikation von undosierten wässrigen Formulierungen auf der Haut beschrieben werden. Beide Veröffentlichungen offenbaren keine Lösung für die flächengenaue Applikation auf die menschliche Haut sowie eine unbehinderte Diffusion wasserlöslicher Wirkstoffe. Ferner wird durch den Stand der Technik keine Lösung für das Problem des Verdunstungsschutzes angegeben. Komplexe pharmazeutische Zubereitungen, die flüchtige Inhaltsstoffe, insbesondere Wasser enthalten, leiden auf der Haut darunter, dass sich durch Verdunsten des flüchtigen Anteils unplanbare Veränderungen in der Formulierung ergeben. Durch bloßes Auftragen auf die Haut oder gegebenenfalls die Applikation von reinen Folien wird das Problem daher nicht umgangen, da die flüssigen wirkstoffhaltigen Formulierungen sich auf der Haut unkontrolliert ausbreiten, damit die Wirkfläche vergrößern und darüber hinaus keine Fixierung des Wirkstoffs auf der Haut gewährleistet ist.

Es galt über längere Zeit als gesicherte Tatsache, dass das Vorhandensein einer impermeablen (okklusiven) Rückschicht in transdermalen therapeutischen Systemen die Hautpermeation von Wirkstoffen generell erhöht (Ann. Red. Med., Vol. 33, 18 (1982); S. 475, 476, US Pat. 4 597 961 (1986), Spalte 2, Zeilen 61-65). In neueren Veröffentlichungen wurde jedoch gezeigt, dass unter okklusiven Bedingungen die Penetration bzw. Permeation der menschlichen Haut nur durch lipophile Wirkstoffe erhöht wird, diejenige durch hydrophile und leicht lipophile Wirkstoffe jedoch unverändert bleibt (Bucks, D.A. et al, J. Invest Dermatol 1988 Jul; 91 (1): 29-33; Treffel P.; Skin Pharmacol. 1992, 5 (2), 108-113). Dokument US 5,629,014 zeigt transdermales therapeutisches System zur kontrollierten Abgabe von Wirkstoffen, umfassend eine Schwammschicht, die einen Wirkstoff enthalt, eine okklusive Ruckschicht und ein Mittel zur Befestigung der Vorrichtung an der Haut.

Überraschenderweise wurde nun gefunden, dass das erfindungsgemäße transdermale therapeutische System, umfassend einen durchgehenden, konzentrisch angeordneten wasserunlöslichen Klebeschichtrand, eine okklusiv wirkende für den Wirkstoff Adrenalin impermeable Rückschicht, eine der Haut zugewandte Vorrichtung zur Abgabe eines hydrophilen Wirkstoffs aus einer wässrigen Phase und eine ablösbare Schutzfolie, die kontrollierte Freisetzung des Wirkstoffs Adrenalin aus der Abgabevorrichtung und eine gesteigerte Permeation durch die Haut ermöglicht.

Das erfindungsgemäße transdermale therapeutische System kann im einzelnen wie folgt beschrieben werden:
Die zentrale Vorrichtung zur Abgabe des Wirkstoffs Adrenalin besteht aus zwei Phasen, wobei die stationäre feste Phase gebildet wird von einem Feststoff, der flexibel sein kann und eine faserförmige oder offenporige vlies- oder schwammartige Struktur aufweist, und wobei die flüssige Phase aus einer den pharmazeutisch aktiven Wirkstoff Adrenalin enthaltenden wässrigen Lösung, Emulsion oder Suspension besteht.

Die zweiphasige Abgabevorrichtung ist konzentrisch umgeben von einem Klebeschichtrand, bestehend aus einem üblichen adhesiven Polymer. In einer bevorzugten Ausgestaltung der Erfindung wird dieser Rand hinsichtlich der Dicke verstärkt durch eine Schicht aus nicht klebenden Polymeren, wobei diese auch in Form von geschlossenporigen Schäumen vorliegen können. Durch die Dicke dieser Schicht, welche in einem Bereich von 200-5000 µm, vorzugsweise von 500-2000 liegt, lässt sich auch der Raum für die zentrale Abgabevorrichtung regulieren.

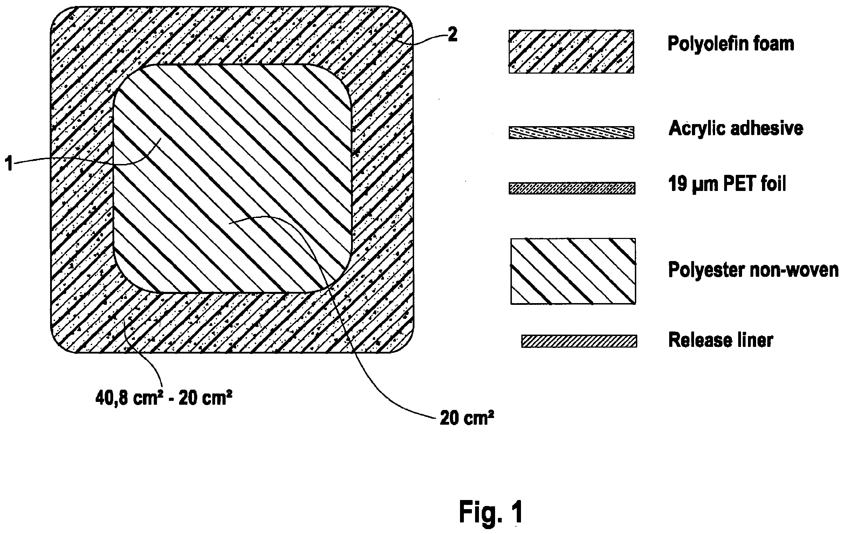

Die Abgabevorrichtung kann kreisförmig sein oder ein Quadrat oder Rechteck darstellen, deren Ecken abgerundet oder abgeschrägt sein können (Fig. 1). Im Falle einer kreisförmigen Abgabevorrichtung hat der Klebeschichtrand eine ringförmige Form.

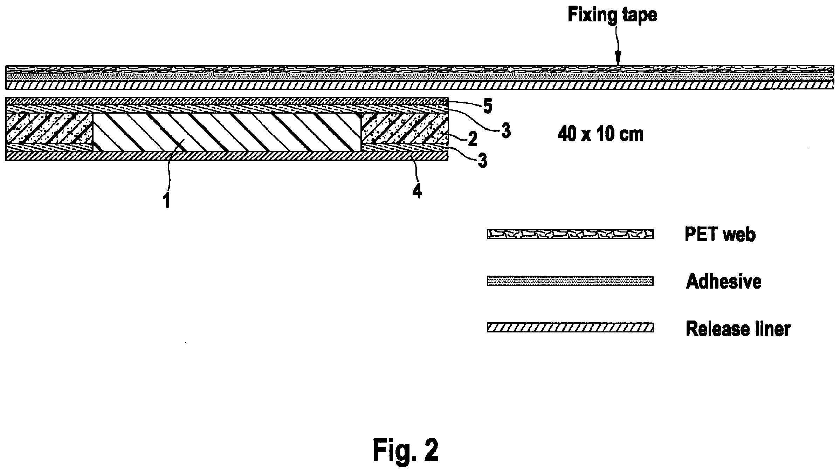

Die vorliegende Erfindung wird durch die Abbildungen Fig. 1 und Fig. 2 näher erläutert.
Fig. 1 zeigt die Aufsicht auf ein erfindungsgemäßes transdermales therapeutisches System.
Fig. 2 zeigt einen Querschnitt durch das erfindungsgemäße System.

1 stellt die zentrale Abgabevorrichtung dar, 2 eine Schicht aus mindestens einem nicht klebenden Polymer, 3 eine Klebschicht, 4 einen Release-Liner und 5 eine okklusive Rückschicht.

Die stationäre Phase der zentralen Abgabevorrichtung ist eine feste Phase, die starr oder flexibel sein kann und eine faserförmige, offenporige, vlies- oder schwammartige Struktur aufweist. Als Materialien für diese feste Phase kommen Stoffe aus der Gruppe der synthetischen oder natürlichen Fasermaterialen, z.B. Cellulose, Viskose, Polyesterfasern, Polyurethanfasern, Silikonfasern und andere in Betracht, bevorzugt sind sogenannte "non-voven"-Materialien, z.B. Vliese.

Als flüssige Phase der zentralen Abgabevorrichtung wird eine wässrige Lösung des hydrophilen, d.h. gut wasserlöslichen pharmazeutischen Wirkstoffs Adrenalin verwendet, wobei diese wässrige Lösung auch Teil einer Emulsion oder Suspension sein kann.

Die flüssige Phase kann zusätzlich zu den Hilfsstoffen, die zur Bildung einer Emulsion oder Suspension erforderlich sind, weitere Hilfsstoffe enthalten, welche z.B. die Bildung von Liposomen ermöglichen.

Als hydrophiler Wirkstoff soll im Kontext dieser Erfindung Adrenalin verstanden werden, der in Wasser eine höhere Löslichkeit aufweist, als in organischen Medien.

Für die okklusive Rückschicht kommen olefinische Folien wie z.B. solche aus Polyethylen, Polypropylenen oder Polyurethanen, bevorzugt jedoch eine Polyethylenterephthalatfolie in Betracht. Der Klebeschichtrand kann aus Polymeren der Polyisopren-, Polyisobutylen oder Polyacrylestergruppe oder auch aus Polysiloxancopolymeren bestehen.

Die Schicht aus nicht klebendem Polymer, die zur Verstärkung des Klebeschichtrandes dient, besteht aus Polyolefinen, bevorzugt aus einem Schaum aus mindestens einem dieser Materialien.

Die folgenden Analog-Beispiele für halbfeste bzw. flüssige wirkstoffhaltige Formulierungen mit einem Peptid dienen der Erläuterung.

**Analog-Beispiel 1 : Eine halbfeste Formulierung**

| | |
|---|---|
| Aqua purificata | 10 g |
| Natrium benzoate | 0,03 g |
| Bile Salts | 0,8 g |
| Cholesterol | 0,7 g |
| Polyoxyethylene | 0,8 g |
| Peptide | 0,1 g |
| SDS | 0,5 g |
| Glycerol | 2,0 g |

**Analog-Beispiel 2: Eine flüssige Formulierung**

| | |
|---|---|
| Aqua purificata | 10 g |
| Parahydroxybenzoeacid (PHB) | 0,03 g |
| Peptide | 0,1 g |
| Marcrogol | 0,8 g |
| Sorbitanmonostearat | 0,8 g |
| Triglyceride medium chain | 0,5 g |
| Glycerol | 1,0 g |

## Patentansprüche

1. Transdermales therapeutisches System zur kontrollierten Abgabe eines wasserlöslichen, pharmazeutischen Wirkstoffs aus einer wässrigen Phase, umfassend eine okklusive Rückschicht, eine der Haut zugewandte zentrale Vorrichtung zur Abgabe des Wirkstoffs, eine die Abgabevorrichtung konzentrisch umgebende Klebeschicht sowie eine wiederablösbare Schutzfolie, wobei der wasserlösliche pharmazeutische Wirkstoff Adrenalin ist, **dadurch gekennzeichnet, dass** die genannte Vorrichtung aus einer stationären festen Phase und einer den Wirkstoff in wässriger Lösung enthaltenden flüssigen Phase besteht, wobei die feste Phase gebildet wird von einem Feststoff, der eine vlies- oder schwammartige Struktur aufweist und aus mindestens einem synthetischen und/oder natürlichen Fasermaterial, ausgewählt aus Cellulose, Viskose, Polyesterfasern, Polyurethanfasern und Silikonfasern, besteht.

2. Transdermales therapeutisches System gemäß Anspruch 1, **dadurch gekennzeichnet, dass** die feste Phase der zentralen Abgabevorrichtung starr oder flexibel ist.

3. Transdermales therapeutisches System gemäß Anspruch 1 und 2, **dadurch gekennzeichnet, dass** die feste Phase eine faserförmige und/oder offenporige, vliesoder schwammartige Struktur aufweist.

4. Transdermales therapeutisches System gemäß mindestens einem der vorhergehenden Ansprüche, **dadurch gekennzeichnet, dass** die feste Phase aus einem Vlies besteht.

5. Transdermales therapeutisches System, gemäß mindestens einem der vorhergehenden Ansprüchen, **dadurch gekennzeichnet, dass** die flüssige Phase der zentralen Abgabevorrichtung aus einer wässrigen Lösung eines pharmazeutischen Wirkstoffes besteht.

6. Transdermales therapeutisches System gemäß mindestens einem der vorhergehenden Ansprüche, **dadurch gekennzeichnet, dass** die flüssige Phase der zentralen Abgabevorrichtung gebildet wird von einer die wässrige Lösung des pharmazeutischen Wirkstoffs enthaltenden Suspension oder Emulsion.

7. Transdermales therapeutisches System gemäß mindestens einem der vorhergehenden Ansprüche, **dadurch gekennzeichnet, dass** die okklusive Rückschicht für den pharmazeutischen Wirkstoff impermeabel ist.

8. Transderrnaies therapeutisches System gemäß mindestens einem der vorhergehenden Ansprüche, **dadurch gekennzeichnet, dass** die die zentrale Abgabevorrichtung konzentrisch umgebende Klebeschicht aus einem adhäsiven Polymer besteht.

9. Transdermales therapeutisches System gemäß Anspruch 8, **dadurch gekennzeichnet, dass** die Klebeschicht zur Erhöhung der Dicke durch mindestens eine Schicht aus nicht klebenden Polymeren verstärkt ist.

## Claims

1. Transdermal therapeutic system for controlled delivery of a water-soluble, active pharmaceutical substance from an aqueous phase, comprising an occlusive backing layer, a central device facing the skin and intended for delivery of the active substance, an adhesive layer concentrically surrounding the delivery device, and a redetachable protective foil, wherein the water-soluble active pharmaceutical substance is adrenaline, **characterized in that** said device is composed of a stationary solid phase and a liquid phase which comprises the active substance in aqueous solution, the solid phase being formed by a solid which has a fleece- or spongelike structure and is composed of at least one synthetic and/or natural fiber material selected from cellulose, viscose, polyester fibers, polyurethane fibers, and silicone fibers.

2. Transdermal therapeutic system according to Claim 1, **characterized in that** the solid phase of the central delivery device is rigid or flexible.

3. Transdermal therapeutic system according to Claim 1 and 2, **characterized in that** the solid phase has a fibrous and/or open-pore fleece- or spongelike structure.

4. Transdermal therapeutic system according to at least one of the preceding claims, **characterized in that** the solid phase is composed of a nonwoven.

5. Transdermal therapeutic system according to at least one of the preceding claims, **characterized in that** the liquid phase of the central delivery device is composed of an aqueous solution of an active pharmaceutical substance.

6. Transdermal therapeutic system according to at least one of the preceding claims, **characterized in that** the liquid phase of the central delivery device is formed from an emulsion or suspension which comprises the aqueous solution of the active pharmaceutical substance.

7. Transdermal therapeutic system according to at least one of the preceding claims, **characterized in that** the occlusive backing layer is impermeable for the active pharmaceutical substance.

8. Transdermal therapeutic system according to at least one of the preceding claims, **characterized in that** the adhesive layer concentrically surrounding the central delivery device is composed of an adhesive polymer.

9. Transdermal therapeutic system according to Claim 8, **characterized in that**, for the purpose of increasing the thickness, the adhesive layer is reinforced by at least one layer of nonadhesive polymers.

## Revendications

1. Système thérapeutique transdermique pour une administration contrôlée d'un agent actif pharmaceutique soluble dans l'eau en phase aqueuse, comprenant une couche arrière occlusive, un dispositif central orienté vers la peau pour une administration de l'agent actif, une couche adhésive entourant de manière concentrique le dispositif d'administration ainsi qu'une pellicule protectrice amovible, dans lequel l'agent actif pharmaceutique soluble dans l'eau est l'adrénaline, **caractérisé en ce que** ledit dispositif consiste en une phase solide stationnaire et une phase liquide contenant l'agent actif en solution aqueuse, dans lequel la phase solide est formée par un agent solide qui présente une structure de non-tissé ou spongieuse et est sélectionné à partir d'au moins un matériau en fibres synthétiques et/ou naturelles, sélectionné à partir des celluloses, des viscoses, des fibres de polyester, des fibres de polyuréthane et des fibres de silicone.

2. Système thérapeutique transdermique selon la revendication 1, **caractérisé en ce que** la phase solide du dispositif d'administration central est rigide ou souple.

3. Système thérapeutique transdermique selon les revendications 1 et 2, **caractérisé en ce que** la phase solide présente une structure fibreuse et/ou à pores ouverts, de non-tissé ou spongieuse.

4. Système thérapeutique transdermique selon au moins l'une des revendications précédentes, **caractérisé en ce que** la phase solide consiste en un non-tissé.

5. Système thérapeutique transdermique selon au moins l'une des revendications précédentes, **caractérisé en ce que** la phase liquide du dispositif d'administration central consiste en une solution aqueuse d'un agent actif pharmaceutique.

6. Système thérapeutique transdermique selon au moins l'une des revendications précédentes, **caractérisé en ce que** la phase liquide du dispositif d'administration central est formée par une suspension ou une émulsion contenant la solution aqueuse de l'agent actif pharmaceutique.

7. Système thérapeutique transdermique selon au moins l'une des revendications précédentes, **caractérisé en ce que** la couche arrière occlusive est imperméable à l'agent actif pharmaceutique.

8. Système thérapeutique transdermique selon au moins l'une des revendications précédentes, **caractérisé en ce que** le dispositif d'administration central consiste en une couche adhésive en polymère adhésif entourant de manière concentrique le dispositif d'administration central.

9. Système thérapeutique transdermique selon la revendication 8, **caractérisé en ce que** la couche adhésive est renforcée pour l'augmentation de l'épaisseur par le biais d'au moins une couche en polymères non adhésifs.
